Europäisches Patentamt

(19) European Patent Office

Office européen des brevets

(11) Veröffentlichungsnummer: **0 102 648
B1**

(12) **EUROPÄISCHE PATENTSCHRIFT**

(45) Veröffentlichungstag der Patentschrift:
07.01.87

(51) Int. Cl.⁴: **A 61 B 6/04,** A 61 G 1/02

(21) Anmeldenummer: 83108824.0

(22) Anmeldetag: 07.09.83

(54) Tisch zur Lagerung von Patienten.

(30) Priorität: 08.09.82 DE 3233363

(43) Veröffentlichungstag der Anmeldung:
14.03.84 Patentblatt 84/11

(45) Bekanntmachung des Hinweises auf die Patenterteilung:
07.01.87 Patentblatt 87/2

(84) Benannte Vertragsstaaten:
AT BE CH DE FR GB IT LI LU

(56) Entgegenhaltungen:
EP - A - 0 047 958
DE - A - 2 308 214
DE - A - 2 410 420
DE - A - 3 138 326
DE - A - 3 146 234
DE - U - 7 030 791
GB - A - 2 068 301
US - A - 3 820 176

(73) Patentinhaber: Stierlen-Maquet Aktiengesellschaft,
Kehler Strasse 31, D-7550 Rastatt (DE)

(72) Erfinder: Schnelle, Eberhard, Dr., Baldenaustrasse 18,
D-7550 Rastatt (DE)
Erfinder: Greiner, Hans, Stadtwaldstrasse 5a,
D-7554 Kuppenheim (DE)
Erfinder: Noth, Siegmar, Brunnenstrasse 24,
D-7502 Malsch-Völkersbach (DE)
Erfinder: Peter, Dieter, Hornisgrindestrasse 4,
D-7570 Baden-Baden 24 (DE)

(74) Vertreter: Patentanwälte Schaumburg & Thoenes,
Mauerkircherstrasse 31 Postfach 86 07 48,
D-8000 München 80 (DE)

## Beschreibung

Die Erfindung bezieht sich auf einen Tisch zur Lagerung von Patienten nach dem Oberbegriff des Anspruchs 1. Ein Tisch dieser Art ist in der US-A-3 820 176 beschrieben.

Der Erfindung liegt die Aufgabe zugrunde, einen Tisch der eingangs genannten Art derart weiterzubilden, dass er leicht handhabbar ist, insbesondere bei der Umbettung von Patienten auf die Plattenanordnung oder von dieser herunter, sowie bei einer erforderlich werdenden Reinigung.

Die Aufgabe wird gemäss der Erfindung bei einem Tisch zur Lagerung von Patienten der eingangs genannten Art durch die im kennzeichnenden Teil des Anspruchs 1 angegebenen Merkmale gelöst.

Ausgestaltungen der Erfindung sind in den Unteransprüchen angegeben.

Die Erfindung wird im folgenden anhand der Zeichnungen näher erläutert, in denen ein Ausführungsbeispiel dargestellt ist. Es zeigt:

Fig. 1 einen Patientenlagerungstisch in Seitenansicht;

Fig. 2 eine Draufsicht auf die Plattenanordnung des Tisches gemäss Fig. 1;

Fig. 3 einen teilweisen Querschnitt durch die Plattenanordnung entsprechend der Linie III-III in Fig. 2;

Fig. 4 einen teilweisen Längsschnitt durch den Bereich einer Stirnseite der Plattenanordnung gemäss der Linie IV-IV in Fig. 2;

Fig. 5 einen teilweisen Querschnitt durch den Tisch gemäss Fig. 1 im Bereich eines Trageholmes entsprechend der Linie V A-V A in Fig. 2 und der Linie V B-V B in Fig. 6;

Fig. 6 eine gegenüber Fig. 1 vergrösserte, ausschnittweise Seitenansicht im Bereich eines Trageholmes;

Fig. 7 einen teilweisen Querschnitt durch die Plattenanordnung im Bereich der Anlenkstelle von deren Kopfplatte entsprechend der Linie VII-VII in Fig. 2;

Fig. 8 einen teilweisen Längsschnitt im Bereich derselben Anlenkstelle entsprechend der Linie VIII-VIII in Fig. 2;

Fig. 9 einen teilweisen Querschnitt durch die Plattenanordnung im Bereich der Anlenkstelle von Stützen an der Kopfplatte gemäss der Linie IX-IX in Fig. 2;

Fig. 10 einen teilweisen Längsschnitt im selben Bereich wie Fig. 9 entsprechend der Linie X-X in Fig. 2;

Fig. 11 eine Stirnansicht des Gestells des Tisches bei Blick in Fig. 1 von links her;

Fig. 12 eine Draufsicht auf das Gestell des Tisches gemäss Fig. 1;

Fig. 13 eine gegenüber Fig. 11 vergrösserte, teilweise geschnittene Stirnansicht des Gestells des Tisches.

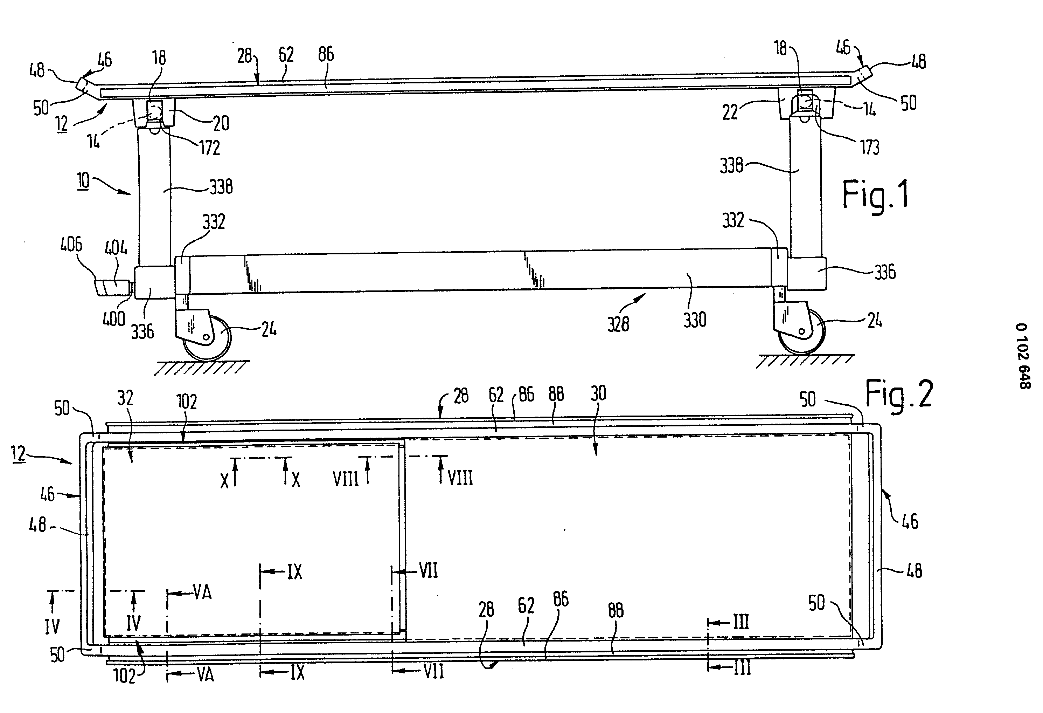

Der zur Lagerung von Patienten dienende Tisch umfasst ein Gestell 10 (Fig. 1, 11, 12) und eine darauf lösbar gehaltene Plattenanordnung 12 (Fig. 1, 2). Das Gestell 10 weist zwei nahe seinen Stirnseiten quer zu seiner Längserstreckung verlaufende, waagerechte Trageholme 14 auf, die jeweils von einem im Querschnitt kreisrunden Rohr 16 und zwei an dessen Enden befestigten, über das Rohr 16 hinaus nach oben ragenden Wangen 18 gebildet sind.

Letztere halten die Plattenanordnung 12 an deren Stützblöcken 20, 22 gegen eine seitliche Verschiebung gegenüber dem Gestell 10. Das Gestell 10 ist mittels vier Nachlaufrollen 24 fahrbar ausgebildet. Mittels eines Fusstritt-Doppelhebels 26, der in drei Stellungen verrastbar ist, sind die Nachlaufrollen 24 entweder gegen Drehung und Verschwenkung um die Hochachse blockiert oder nur gegen eine Verschwenkung blockiert, aber drehbar oder aber in einer dritten Stellung frei drehbar und verschwenkbar.

Die Plattenanordnung 12 ist aufgrund ihrer nachstehend beschriebenen Konstruktion sehr stabil und insbesondere verwindungssteif ausgebildet, so dass sie auch in vom Gestell 10 getrenntem Zustand bei Einsätzen aller Art verwendbar ist. Sie umfasst zwei spiegelsymmetrisch bezüglich der Längsachse ausgebildete, aufgrund ihrer noch zu beschreibenden Profilierung besonders biegesteife Längsholme 28, die ihre Längsseiten bilden, und zwei zwischen den Längsholmen 28 gehaltene Platten, nämlich eine Beinplatte 30 und eine Kopfplatte 32. Letztere ist in noch zu beschreibender Weise nahe der Beinplatte 30 schwenkbar an den Längsholmen 28 angelenkt, so dass sie ausgehend von einer mit der Beinplatte fluchtenden, bei auf dem Gestell wie in Fig. 1 waagerecht gehaltener Plattenanordnung 12 ebenfalls waagerechter Stellung, in der sie zwischen den Längsholmen 28 liegt, derart nach oben verschwenkt werden kann, dass ihr der Beinplatte 30 abgewandtes Kopfende höher als die Beinplatte 30 liegt. Dabei ist sie in unterschiedlichen Schwenkwinkeln arretierbar. Zweckmässig betragen die Länge der Beinplatte 30 wie beim Ausführungsbeispiel zumindest annähernd 60% und die Länge der Kopfplatte 32 zumindest annähernd 40% der Gesamtlänge beider unter Belassung nur eines geringen Zwischenraumes aneinander anschliessender Platten 30, 32. Bei dieser Bemessung dient die Beinplatte 30 zur Lagerung der Beine und auch des Unterkörpers eines Patienten, während die Kopfplatte 32 zur Lagerung des Oberkörpers und Kopfes dient. Der maximale Schwenkwinkel der Kopfplatte 32 sollte hierbei zweckmässig bei 65° liegen, um den Patienten mit seinem Oberkörper weitgehend aufrichten zu können.

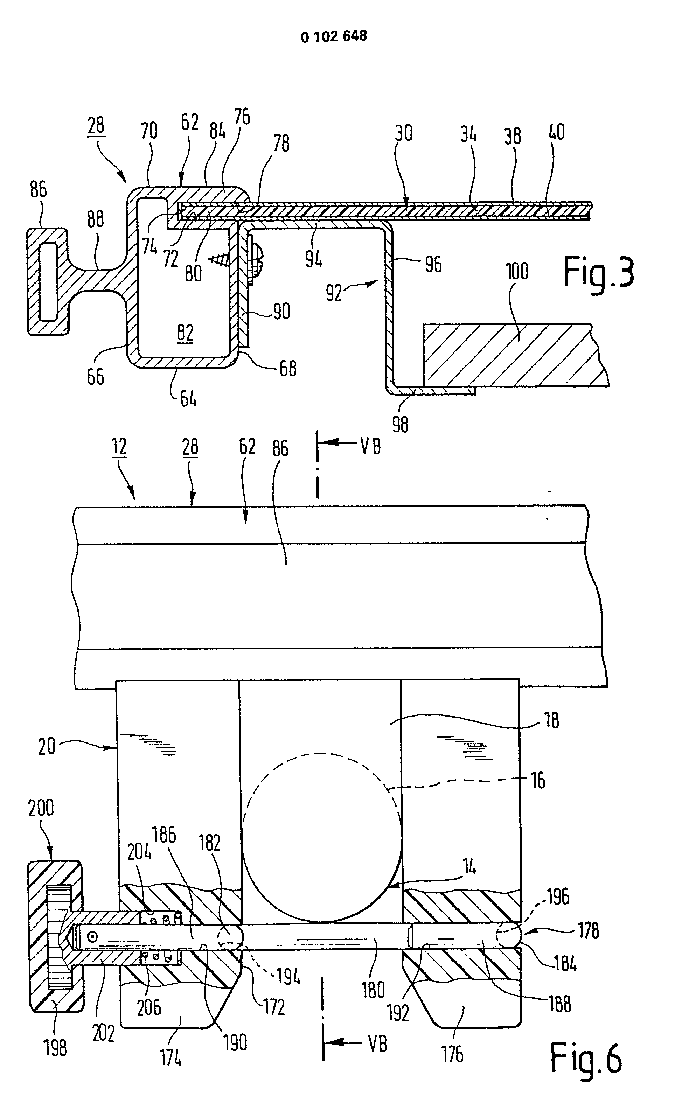

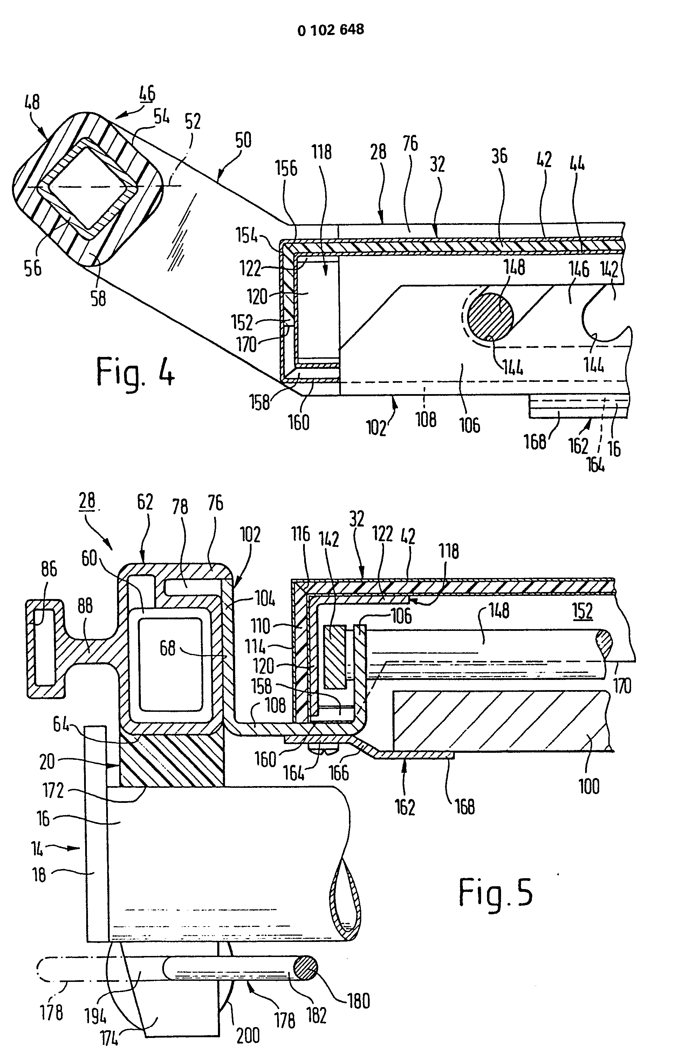

Die Beinplatte 30 und die Kopfplatte 32 sind für Röntgenaufnahmen durchleuchtbar ausgebildet. Um die Röntgenstrahlen möglichst wenig zu schwächen und auch aus Gründen der Gewichtsersparnis sollen die Platten 30, 32 möglichst dünn ausgebildet sein, während andererseits eine gute Festigkeit wünschenswert ist, damit sich die Platten 30, 32 nicht unter dem Gewicht eines Patienten durchbiegen und damit sie zur Verwindungssteifheit der Plattenanordnung 12 beitragen. Es wurde gefunden, dass derartige Eigenschaften der Platten 30, 32 abweichend von bekannten, aus einer Kunststoff-Grundplatte und einer Polsterung bestehenden Platten dadurch erreicht werden können, dass die Platten 30, 32, wie beispielsweise in Fig. 3 und 4 sichtbar, aus einer relativ dicken Kunststoffschicht 34, 36 und mindestens einer flächig damit verbundenen, gegenüber der Kunststoffschicht 34, 36 dünnen Metallschicht 38, 40 bzw. 42, 44 bestehen. Besonders günstig ist es, wenn die Kunststoffschicht 34 beidseitig mit solchen Metallschichten 38, 40 bzw. 42, 44 versehen ist. Diese können mit der Kunststoffschicht 34 bzw. 36 verklebt sein. Als geeignetes Material für die Kunststoffschicht 34, 36 hat sich Polyäthylen erwiesen. Die Metallschichten 38, 40; 42, 44 bestehen zweckmässig wie beim Ausführungsbeispiel aus Aluminium. Hierbei kann es sich insbesondere um ein hartes, allerdings nicht sprödes, aussenseitig eloxiertes Aluminium handeln. Bei diesem Schichtaufbau der Platten 30, 32 genügt es, wenn diese jeweils eine Gesamtdicke von 4 mm haben, die sich beim Ausführungsbeispiel zusammensetzt aus einer Dicke von 3 mm der Kunststoffschicht 36, 38 und Dicken von 0,5 mm der Metallschichten 38, 40 bzw. 42, 44. Jedenfalls sollten die Beinplatte 30 und die Kopfplatte 32 untereinander gleiche Dikken und gleichen Schichtaufbau haben, damit sie bei einer Durchleuchtung gleiche Absorptionscharakteristiken aufweisen.

Wie insbesondere aus Fig. 1, 2 und 4 erkennbar ist, sind die Längsholme 28 an den Stirnseiten der Plattenanordnung mittels Querholmen 46 verbunden, die mit den Längsholmen 28 einen rechteckigen, verwindungssteifen Rahmen bilden. Die Querholme 46 haben eine in der Draufsicht U-förmige Gestalt mit einem geraden, senkrecht zu den Querholmen 28 verlaufenden Griffabschnitt 48 und zwei daran zu den Längsholmen 28 hin abgebogenen und an deren Enden befestigten Schenkeln 50. Der Griffabschnitt verläuft von der jeweils benachbarten Stirnseite der Beinplatte 30 bzw. der Kopfplatte 32 derart beabstandet, dass er von einer Bedienungsperson leicht umgriffen werden kann. Ebenfalls aus ergonomischen Gründen sowie um einem Herabrutschen eines liegenden Patienten von der Plattenanordnung 12 in deren Längsrichtung entgegen zu wirken, haben die Schenkel 50 einen gegenüber der Ebene der Längsholme 28 von diesen fort nach oben gebogenen Verlauf, so dass der Griffabschnitt 48 mit mehr als der Hälfte seiner Höhe oberhalb der Oberseite der Längsholme 28 liegt. Sowohl aus ergonomischen Gründen als auch um eine Verletzung eines an einen Griffabschnitt 48 anstossenden Patienten zu vermeiden, ist der Griffabschnitt 48 derart angeordnet, dass seine eine, in Fig. 4 waagerechte Diagonale 52 parallel zur Ebene der Längsholme 28 verläuft. Dadurch hat eine zur Beinplatte 30 bzw. zur Kopfplatte 32 hin liegende, oben liegende Seite 54 des Griffabschnittes 48 eine gegenüber dem Patienten sanfte Neigung. Zudem ist der Griffabschnitt 48 an den Ecken seines Querschnittes stark gerundet. Der gesamte Querholm besteht jeweils aus einem rechteckigen und im Ausführungsbeispiel im Griffabschnitt 48 quadratischen, metallischen Rohr 56 und einer Ummantelung 58 aus Polyurethanschaum. Die Schenkel 50 setzen sich in Zapfen 60 (Fig. 5) fort, die in die Enden der hohlen Längsholme 28 eingesteckt und darin befestigt sind. Die Ummantelung 58 erstreckt sich unter Freilassung der Zapfen 60 über sowohl den Griffabschnitt 48 wie auch die Schenkel 50 und schliesst äusserlich mit der Oberseite, Aussenseite und Unterseite eines zumindest annähernd rechteckigen Querschnittsbereiches 62 (Fig. 3, 5) fluchtend an die Enden der Längsholme 28 an.

Der auch in den Figuren 5, 7 und 9 sichtbare Querschnitt der spiegelbildlich zueinander ausgebildeten Längsholme 28 sei anhand von Fig. 3 erläutert. Der bereits erwähnte, annähernd rechteckige Hauptquerschnittsbereich 62 eines Längsholmes 28 weist eine liegende Unterseite 64, eine an die Unterseite 64 anschliessende, zu ihr senkrechte Aussenseite 66, eine an die Unterseite 64 anschliessende, zu ihr senkrechte und somit zur Aussenseite parallele, jedoch ihr gegenüber weniger hohe erste Innenseite 68, eine an die Aussenseite 66 anschliessende, zur Unterseite parallele erste Oberseite 70, eine an die erste Innenseite 68 anschliessende, zur Unterseite 64 parallele zweite Oberseite 72, eine an diese anschliessend nach oben ragende zweite Innenseite 74 sowie eine Lippe 76 auf. Letztere erstreckt sich, ausgehend von der zweiten Innenseite 74, unter Belassung einer Nut 78 beabstandet von der zweiten Oberseite 72 parallel zu dieser. Die Nut 78 dient zur Aufnahme eines Längsrandes 80 der Beinplatte 30, die beiderseits in dieser Weise gehalten ist. Die Nut 78 hat hierfür eine der Dicke der Beinplatte 30 gleiche Weite. In der Nut 78 ist der jeweilige Längsrand 80 verklebt. Zur Erzielung einer möglichst guten Verbindungswirkung beträgt die Breite der zweiten Oberseite 72 und damit die Tiefe der Nut 78 zweckmässig wie beim Ausführungsbeispiel mehr als 50% der Breite der Unterseite 64. Da der Hauptquerschnittsbereich 62 hohl ist, wobei sein Innenraum 82 von den die Unterseite 64, die Aussenseite 66, die ersten und zweiten Innenseiten 68, 74 und die ersten und zweiten Oberseiten 70, 72 bildenden Wandungen ununterbrochen umschlossen ist, hat er bei geringem Gewicht bereits eine hohe Biegesteifigkeit und Verwindungssteifheit. Die Lippe 76 erstreckt sich zweckmässig zur Förderung einer guten Verbindung mit dem Längsrand 80 mindestens bis zur Ebene der Innenseite 68, und zur Förderung der erwähnten Festigkeitseigenschaften sollte sie so hoch am Hauptquerschnittsbereich 62 sitzen, dass ihre Oberseite 84 mit der ersten Oberseite 70 des Hauptquerschnittsbereiches 62 fluchtet.

Parallel zu den Aussenseiten 66 und von diesen beabstandet verlaufen Schienen 86 von stehendrechteckigem Querschnitt, die in üblicher Weise zur Befestigung von Hilfsgeräten wie Bluttransfusionsapparaten dienen können und die hier eine zusätzliche Versteifung der Längsholme 28 gegen Durchbiegung und Verwindung bilden, da sie mit den Hauptquerschnittsbereichen 62 verbunden sind. Auch die Schienen 86 haben einen Hohlquerschnitt. Sie sind annähernd in ihrer halben Höhe mit den Hauptquerschnittsbereichen 62 über senkrecht zu deren Aussenseiten 66 verlaufende Verbindungselemente verbunden; als solche Verbindungselemente sind parallel zu den Unterseiten 64 der Hauptquerschnittsbereiche 62 verlaufende, einstückig mit jeweils einer Schiene 86 und einem Hauptquerschnittsbereich 62 gebildete Stege 88 vorgesehen. Diese treffen die Aussenseiten 66 der Hauptquerschnittsbereiche 62 annähernd in deren halber Höhe.

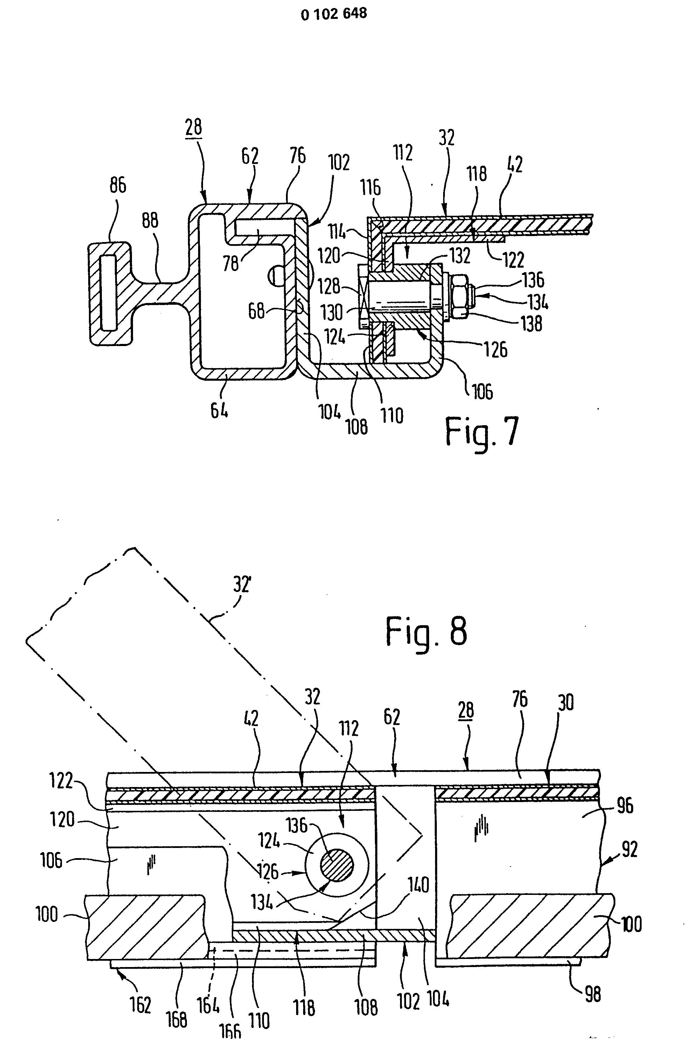

Wie aus Fig. 3 und 8 ersichtlich ist, ist an den Innenseiten 68 (Fig. 3) der Hauptquerschnittsbereiche 62 jeweils ein Schenkel 90 eines Winkelprofils 92 anliegend befestigt, das mit einem von diesem Schenkel 90 senkrecht abgebogenen oberen Schenkel 94 an der Unterseite der Beinplatte 30 anliegt und daran durch Verkleben befestigt ist. Das Winkelprofil 92 erstreckt sich annähernd über die Länge der in den Nuten 78 der Längsholme 28 gehaltenen Beinplatte 30. Jedes der beiden Winkelprofile 92 weist weiter einen von seinem oberen Schenkel 94 senkrecht nach unten abgebogenen Schenkel 96 und einen von dessen unterem Ende von dem benachbarten Längsholm 28 fort und parallel zur Beinplatte 30 einwärts gebogenen unteren Schenkel 98 auf. Der geringste gegenseitige Abstand der nach unten abgebogenen Schenkel 96 der Winkelprofile 92 gleicht annähernd der Breite einer oder mehrerer von den Stirnseiten der Plattenanordnung 12 her auf die unteren Schenkel 98 einschiebbaren Röntgenfilmkassetten 100. Um deren Einschub von der Stirnseite der Beinplatte 30 her zu erleichtern, endet das Winkelprofil 92 in einem Abstand von 50 mm von der genannten Stirnseite.

Wie insbesondere aus den Figuren 2, 4, 5 und 7 bis 10 ersichtlich ist, tragen die Längsholme 28 annähernd über die Länge der Kopfplatte 32 auf ihren Innenseiten 68 jeweils eine Profilleiste 102, die einen an der Innenseite 68 des benachbarten Längsholmes 28 anliegend befestigten äusseren Schenkel 104 aufweist und an der die Kopfplatte 32 schwenkbar angelenkt ist. Dabei überragt die Lippe 76 die Innenseite 68 um die Dicke dieses äusseren Schenkels 104, und dieser äussere Schenkel 104 stösst an die Unterseite der Lippe 76 an, so dass im Bereich der Kopfplatte 32 die Nut 78 gegen ein Eindringen von Schmutz verschlossen ist. Gleichzeitig bewirkt der äussere Schenkel 104 ebenso wie die gesamte übrige Profilleiste 102 eine weitere Versteifung des jeweiligen Längsholmes 28.

Die Profilleisten 102 weisen jeweils einen U-förmigen Querschnitt mit einem parallel zu dem äusseren Schenkel 104 in einem Abstand von diesem verlaufenden inneren Schenkel 106 und einem den äusseren und inneren Schenkel 104, 106 verbindenden Jochbereich 108 auf. Die Unterseite des eben ausgebildeten Jochbereiches 108 fluchtet mit den Unterseiten 64 der Längsholme 28.

Um eine Durchleuchtung zu Röntgenzwecken nicht zu behindern, ist die Kopfplatte 32 unter Vermeidung einer zwischen den inneren Schenkeln 106 der Profilleisten 102 durchlaufenden Achse oder Welle ausschliesslich an den inneren Schenkeln 106 der Profilleisten 102 angelenkt. Die Oberkanten der inneren Schenkel 106 der Profilleisten 102 liegen bei mit der Beinplatte 30 fluchtender Kopfplatte 32 höchstens so hoch wie deren Unterseite und beim Ausführungsbeispiel etwas tiefer. Die Breite der Kopfplatte 32 ist grösser als der Abstand zwischen den inneren Schenkeln 106 beider Profilleisten 102, jedoch geringer als der Innenabstand von deren äusseren Schenkeln 104. Von den Längsrändern der Kopfplatte 32 erstrecken sich zumindest im Bereich von deren in Fig. 7 und 8 dargestellter Anlenkstelle an den inneren Schenkeln 106 Plattenränder 110 senkrecht nach unten, die jeweils bei mit der Beinplatte 30 fluchtender Kopfplatte 32 zwischen den inneren und äusseren Schenkeln 106, 104 einer Profilleiste 102 eintauchen und annähernd in der Mitte zwischen diesen liegen. Diese Plattenränder 110 sind über Schwenkgelenke 112 mit den inneren Schenkeln 106 verbunden.

Zur Förderung der Festigkeit der Verbindung zwischen der Kopfplatte 32 und den sich von ihr senkrecht nach unten erstreckenden Plattenrändern 110 weisen letztere eine einstückig mit der oben liegenden Metallschicht 42 der Kopfplatte 32 gebildete äussere Metallschicht 114 auf. Kopfplatte 32 und Plattenränder 114 sind nämlich aus dem Material der Kopfplatte 32 durch rückseitiges Einschneiden einer V-Nut von 90° und Abbiegen der oberen Metallschicht 42 gebildet, wobei die ursprünglichen Nutflächen an einer Stossstelle 116 miteinander verklebt sind. Weiter sind die sich von den Längsrändern der Kopfplatte 32 nach unten erstreckenden Plattenränder 110 jeweils mittels einer Winkelschiene 118 versteift, deren einer Schenkel 120 an der Innenseite eines Plattenrandes 110 anliegend befestigt ist und deren oberer, oberhalb des inneren Schenkels 106 einer Profilleiste 102 liegender Schenkel 122 an der Unterseite der Kopfplatte 32 anliegend befestigt ist. Diese Befestigung erfolgt zweckmässig wie beim Ausführungsbeispiel durch Verklebung.

Die Schwenkgelenke 112 (Fig. 7, 8) umfassen jeweils eine mit einem Bund 124 versehene, einen Plattenrand 110 durchsetzende, mit dem Bund 124 an dessen Innenseite anliegende und an dem inneren Schenkel 106 einer Profilleiste 102 abgestützte Hülse 126. Weiter weisen die Schwenkgelenke 112 jeweils einen mit seinem Kopf 128 auf der Aussenseite eines Plattenrandes 110 liegenden und mit einem zylindrischen Abschnitt 130 eine zentrale Bohrung 132 der Hülse 126 durchsetzenden Bolzen 134 auf, dessen zylindrischer Abschnitt 130 am inneren Schenkel 106 der Profilleiste 102 abgestützt und befestigt ist, während sein Kopf 128 auch am äusseren Ende der Hülse 126 anliegt, so dass der jeweilige Plattenrand 110 zwischen dem Kopf 128 und dem Bund 124 mit vernachlässigbar geringem axialem Spiel verschwenkbar gehalten ist. Ein gegenüber dem zylindrischen Abschnitt 130 dünnerer Gewindeabschnitt 136 des Bolzens 134 durchsetzt den jeweiligen inneren Schenkel 106 der Profilleiste 102 und ist an dessen Innenseite mit einer Mutter 138 verschraubt.

Wie aus einem Vergleich der Fig. 7 mit den Fig. 5, 8 und 9 ersichtlich ist, erstrecken sich beim Ausführungsbeispiel die von den Längsrändern der Kopfplatte 32 nach unten erstreckenden Plattenränder 110 über die Länge der Kopfplatte 32 bei mit der Beinplatte 30 fluchtender Kopfplatte 32 bis zum Aufsitzen auf der Oberseite des Jochbereiches 108. Hierdurch ist dann die Kopfplatte 32 auf den Profilleisten 102 getragen. Damit die Plattenränder 110 ein Hochschwenken der Kopfplatte 32 beispielsweise in die in Fig. 8 bei 32' angedeutete Stellung nicht behindern, weisen sie an ihren zur Beinplatte 30 hin liegenden Enden, von einer Stelle unterhalb des Schwenkgelenks 112 beginnend, eine Abschrägung 140 (Fig. 8) auf.

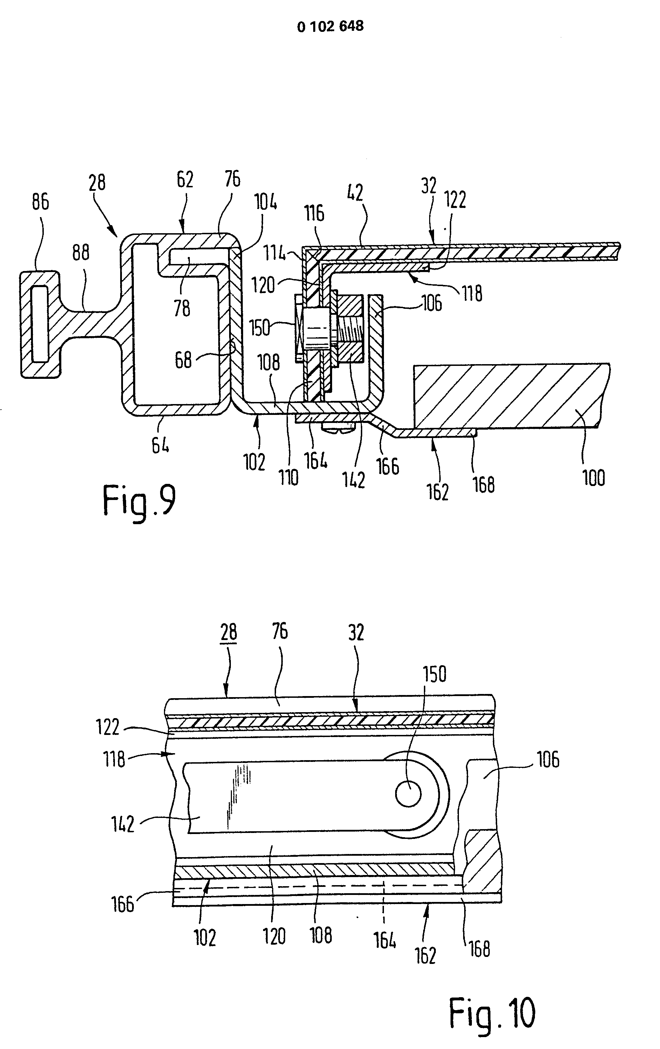

Damit die Kopfplatte 32 in ihrer hochgeschwenkten Stellung arretiert werden kann, ist sie mit zwei annähernd in der Mitte ihrer Längserstreckung nahe ihren Längsrändern und unterhalb ihrer Oberseite angelenkten Stützen 142 (Fig. 4, 5, 9, 10) versehen. Die inneren Schenkel 106 der Profilleisten 102 sind im Bereich zwischen ihren der Beinplatte 30 abgewandten Enden und der in Fig. 9 und 10 dargestellten Anlenkstelle der Stützen 142 mit Rasteinschnitten 144 (Fig. 4) und zwischen diesen gebildeten, in Richtung zur Beinplatte 30 hin geneigten Rastnocken 146 versehen. An ihren freien Enden tragen die Stützen 142 jeweils mindestens ein mit den Rasteinschnitten 144 zusammenwirkendes Rastglied. Dieses ist beim Ausführungsbeispiel von einer die freien Enden der Stützen 142 verbindenden Stange 148 (Fig. 4, 5) gebildet.

Die Stützen 142 sind mittels in sie eingeschraubter jeweils einen Plattenrand 110 durchsetzender Kopfbolzen 150 (Fig. 9, 10) an den Plattenrändern 110 angelenkt und diesen gegenüber in einem Abstand gehalten, der geringer ist als der Abstand dieser Plattenränder 110 von den inneren Schenkeln 106 der Profilleisten 102, so dass die Stützen 142 bei mit der Beinplatte 30 fluchtender Kopfplatte 32 jeweils zwischen einem, dieser Plattenränder 110 und einem inneren Schenkel 106 einer Profilleiste 102 beabstandet von diesen und parallel zu ihnen verlaufen.

Ebenso wie in nicht näher dargestellter Weise die Beinplatte 30 weist auch die Kopfplatte 32 an einer Stirnseite der Plattenanordnung 12 einen nach unten ragenden, von ihr rechtwinklig abgebogenen, in Fig. 4 und 5 sichtbaren Plattenrand 152 auf. Dieser stirnseitige Plattenrand 152 weist eine einstückig mit der oben liegenden Metallschicht 42 der Kopfplatte gebildete äussere Metallschicht 154 (Fig. 4) auf. Der stirnseitige Plattenrand 152 ist nämlich ähnlich wie die von den Längsrändern der Kopfplatte 32 nach unten ragenden Plattenränder 110 aus dem Material der Kopfplatte 32 durch rückwärtiges Einschneiden einer V-Nut von 90° und Abbiegen der oberen Metallschicht 42 unter Verkleben der ursprünglichen Nutflächen an einer Stossstelle 156 gebildet. Weiter erstreckt sich von der Unterseite des stirnseitigen Randes 152 der Kopfplatte 32 nahe deren Längsseiten jeweils ein Randabschnitt 158 parallel zur Kopfplatte 32 unter diese, der eine unten liegende, einstückig mit der oben liegenden Metallschicht 42 der Kopfplatte 32 und der äusseren Metallschicht 154 des stirnseitigen Plattenrandes 152 gebildete Metallschicht 160 aufweist. Diese Randabschnitte 158 sind in entsprechender Weise wie der stirnseitige Plattenrand 152 der Kopfplatte 32 aus deren Material gebildet. Weiter sind die Randabschnitte 158 an ihren zur benachbarten Längsseite der Kopfplatte 32 hin liegenden Enden jeweils durch Verkleben mit einem sich von einem Längsrand der Kopfplatte 32 nach unten erstreckenden Plattenrand 110 verbunden. Der Plattenrand 152 und die Randabschnitte 158 versteifen die Konstruktion der Kopfplatte 32, und der stirnseitige Plattenrand 152 erleichtert ein Erfassen und Hochschwenken bzw. Absenken der Kopfplatte 32.

Wie aus den Figuren 4, 5 und 8 bis 10 ersichtlich ist, ist an den Profilleisten 102 jeweils eine weitere Leiste 162 gehalten. Diese weist einen an der Unterseite des Jochabschnittes 108 anliegend befestigten ersten Schenkel 164, einen von dessen innerem Ende schräg nach unten und innen abgebogenen zweiten Schenkel 166 und einen von dessen unterem Ende von dem benachbarten Längsholm 28 fort und parallel zur Kopfplatte 32 einwärts gebogenen dritten, unteren Schenkel 168 auf. Der geringste gegenseitige Abstand der nach unten abgebogenen, zweiten Schenkel 166 der beiden Leisten 162 gleicht annähernd der Breite einer von den Stirnseiten der Plattenanordnung 12 her auf die unteren Schenkel 168 der Leiste 162 einschiebbaren Röntgenfilmkassette 100. Zur Erleichterung des Einschiebens von der Stirnseite der Kopfplatte 32 her endet die Leiste 162, wie aus Fig. 4 ersichtlich in einem Abstand von annähernd 60 mm von dieser Stirnseite. Weiter hat der stirnseitige Plattenrand 152 der Kopfplatte 32 zur Erleichterung des Einschiebens eine nach unten offene Ausnehmung, die beiderseits von den Randabschnitten 158 begrenzt ist und deren Oberseite 170 um annähernd die Dicke der Röntgenfilmkassette 100 über den Oberseiten der unteren Schenkel 158 der Leisten 162 liegt, während ihre Breite mindestens der Breite der Röntgenfilmkassette 100 gleicht. Die unteren Schenkel 168 der Leisten 162 liegen andererseits mindestens um die Dicke der Röntgenfilmkassette 100 tiefer als die tiefsten Stellen der Rasteinschnitte 144 (Fig. 4), damit das Einschieben nicht durch die als Rastglied vorgesehene Stange 148 behindert wird. Zweckmässig liegen überdies wie beim Ausführungsbeispiel die unteren Schenkel 98 der Winkelprofile 92 (Fig. 3) bei miteinander fluchtenden Bein- und Kopfplatten 30, 32 auf gleicher Höhe mit den unteren Schenkeln 168 jeweils einer Leiste 162 und fluchten mit diesen, so dass eine oder mehrere Röntgenfilmkassetten 100 von einer Stirnseite der Plattenanordnung 112 zur anderen Stirnseite hindurchgeschoben werden kann.

Wie insbesondere aus Fig. 1, 5 und 6 hervorgeht, sind an den Unterseiten der Längsholme 28 vier von diesen nach unten ragende Stützböcke 20, 22 befestigt. Diese weisen jeweils eine in der Seitenansicht der Fig. 1 annähernd umgekehrt U-förmige Gestalt auf, da sie eine nach unten und in Querrichtung offene Ausnehmung 172 bzw. 173 haben. Jeweils zwei kopfseitige Stützböcke 20 bzw. fussseitige Stützböcke 22 sind, wie Fig. 1 zeigt, in einer mit ihren Ausnehmungen 172 bzw. 173 einen Trageholm 14 aufnehmenden Stellung auf diesen aufsetzbar. Beim Ausführungsbeispiel weisen die im Bereich der Kopfplatte 32 angeordneten Kopfseitigen Stützböcke 20 eine Ausnehmung 172 von einer Weite auf, die dem Durchmesser des Rohres 16 des Trageholmes 14 entspricht, während die im Bereich der Beinplatte 30 (Fig. 2) vorgesehenen Stützböcke 22 eine Ausnehmung 173 von gegenüber dem Rohr 16 annähernd doppelter Weite haben. Hierdurch sichern die kopfseitigen Stützböcke 20 die Plattenanordnung 12 gegen eine Längsverschiebung gegenüber dem Gestell 10, während die fussseitigen Stützböcke 22 eine begrenzte Längsverschiebung zulassen. Dies ist nötig zum Ausgleich von Bewegungen, die sich bei unterschiedlichen Höhenstellungen der Trageholme 14 ergeben, da diese in noch zu beschreibender Weise unabhängig von einander höhenverstellbar sind. Die Ausnehmungen 172 de kopfseitigen Stützböcke 120 verlaufen an ihrem Grunde halbkreisförmig gerundet, während der Grund der Ausnehmung 173 der fussseitigen Stützböcke 22 jeweils am Übergang in die zu den Längsholmen 28 senkrechten Innenseiten der Ausnehmung 173 viertelkreisförmig gerundet verläuft, um beim Aufsetzen der Plattenanordnung 12 auf die Stützholme 14 möglichst Beschädigungen der Stützböcke 20, 22 zu vermeiden. Diese bestehen ebenfalls zur Erzielung einer grossen Robustheit bei geringem Gewicht aus einem hochfesten Kunststoff. Sie können daher auch ohne Gefahr eines merklichen Verschleisses als die Plattenanordnung 12 abstützende Füsse dienen, wenn die Plattenanordnung 12 auf einer unebenen Unterlage, beispielsweise dem Erdboden abgestellt wird.

Zur Verriegelung der Stützböcke 20, 22 gegen ein Abheben der Plattenanordnung 12 von den Trageholmen 14 genügt es grundsätzlich, wenn zwei einander diagonal gegenüber liegende Stützböcke 20, 22 mit geeigneten Verriegelungsvorrichtungen versehen sind. Beim Ausführungsbeispiel ist dies hinsichtlich aller Stützböcke 20, 22 der Fall. Die Verriegelungsvorrichtungen sind untereinander gleichartig ausgebildet, weshalb im folgenden anhand von Fig. 6 lediglich eine Verriegelungsvorrichtung beschrieben werden soll, die an einem kopfseitigen Stützbock 20 vorgesehen ist.

Die Ausnehmung 172 des Stützbockes 20 ist tiefer als die lotrechte Querschnittsabmessung des Trageholmes 14 in dessen vom Stützbock 20 beaufschlagtem Längenabschnitt, d.h. beim Ausführungsbeispiel tiefer als der Durchmesser des Rohrs 16. Der Stützbock 20 überragt daher mit seinen beiderseits der Ausnehmung 172 liegenden Schenkeln 174, 176 den Trageholm 14 nach unten. Die Länge des Trageholmes 14 ist annähernd gleich dem Aussenabstand der beiden in Querrichtung der Plattenanordnung 13 einander gegenüber liegenden, auf demselben Trageholm 14 sitzenden Stützböcke 20, so dass die an den Enden des Trageholmes 14 nach oben ragenden Wangen 18 den Aussenseiten jeweils eines Stützbockes 20 eng benachbart sind. In mindestens einem Schenkel 174, 176 und beim Ausführungsbeispiel in beiden ist unterhalb des Trageholmes 14 ein Bügel 178 gelagert. Dieser umfasst einen geraden Jochabschnitt 180, der sich parallel zu den Längsholmen 28 erstreckt und dessen Länge gleich der Summe der Weite der Ausnehmung 172 und der ebenso wie diese Weite in Längsrichtung der Längsholme 28 gemessenen Dicke desjenigen Schenkels 176 ist, der der benachbarten Stirnseite der Plattenanordnung 12 – im Ausführungsbeispiel der Kopfseite – abgewandt ist. An beide Enden des Jochabschnittes 180 schliessen von diesem rechtwinklig abgebogene, parallel zueinander verlaufende Bügelschenkel 182, 184 an. An deren dem Jochabschnitt 180 abgewandten Enden sind mit einander fluchtende Lagerzapfen 186, 188 nach der benachbarten Stirnseite der Plattenanordnung 12, also bei dem Lagerbock 20 nach der Kopfseite hin abgebogen. Diese sind in miteinander fluchtenden Bohrungen 190, 192 gelagert, die in den Schenkeln 174, 176 annähernd in der Mitte zwischen deren Innen- und Aussenseite gebildet sind und parallel zu den Längsholmen 28 verlaufen. Grundsätzlich würde es genügen, lediglich den Lagerzapfen 186 in dem zur benachbarten Stirnseite der Plattenanordnung 12 hin liegenden Schenkel 174 des Lagerbocks 20 vorzusehen, jedoch erlaubt die doppelte Lagerung eine schwächere Dimensionierung des Bügels 187 und ergibt daher eine Gewichtsersparnis. Mindestens ein Schenkel 174, 176 des mit einem Bügel 178 versehenen Lagerbocks 20 und beim Ausführungsbeispiel dessen beide Schenkel 174, 176 weist auf seiner der benachbarten Stirnseite der Plattenanordnung 12 abgewandten Seite eine senkrecht zur Richtung der Längsholme 28 verlaufende Nut 194 bzw. 196 auf, die über die gesamte jeweilige Seite verläuft und die gegenüber dem Halbmesser der Bügelschenkel 182, 184 grössere Tiefe aufweist. Zweckmässig gleicht diese Tiefe wie beim Ausführungsbeispiel dem Durchmesser der Bügelschenkel 182, 184. Die Bohrungen 190, 192 münden in die Nuten 194, 196. Der Bügel 176 kann daher, wenn er mit jeweils einem Längenabschnitt der Bügelschenkel 182, 184 in den Nuten 194, 196 liegt, nicht verschwenkt werden, während er dann, wenn er aus den Nuten 194, 196 von der benachbarten Stirnseite der Plattenanordnung 12 fort herausgetreten ist, um seine Lagerzapfen 186, 188 verschwenkt werden kann. Dabei ist eine Verschwenkung möglich zwischen einer in Fig. 5 ausgezogen dargestellten Verriegelungsstellung, bei der der Jochabschnitt 180 des Bügels 178 unterhalb des Trageholmes 14 liegt, und einer in Fig. 5 strichpunktiert bei 178' angedeuteten Freigabestellung, bei der der Bügel 178 das äussere Ende des Trageholmes 14 nach aussen überragt. In der Verriegelungsstellung ist dann, wenn die Bügelschenkel 182, 184 in den Nuten 194, 196 liegen, ein Abheben der Plattenanordnung 12 vom Trageholm nicht möglich, da dann der Jochabschnitt 180 an die Unterseite des Trageholmes 14 anschlägt. In der Freigabestellung ist dagegen ein Abheben möglich, da dann der das Ende des Trageholmes 14 umgebende Bügel 178 an dem Ende des Trageholmes 14 vorbei nach oben abgehoben werden kann. Die lichte Weite des Bügels 178 zwischen dessen Bügelschenkeln 182, 184 ist nämlich aufgrund der oben erläuterten Bemessung der Länge des Jochabschnittes 180 jedenfalls grösser als die parallel zu den Längsholmen 28 gemessene Abmessung des Endabschnittes des Trageholmes 14, und die Länge der Bügelschenkel 182, 184 ist grösser als der Abstand der Lagerzapfen 186, 188 von dem benachbarten äusseren Ende des Trageholmes 14.

Um den Bügel 178 in seiner Verriegelungsstellung mit in den Nuten 194, 196 liegenden Bügelschenkeln 182, 184 zu halten, ist der zur benachbarten Stirnseite der Plattenanordnung 12 hin liegende Lagerzapfen 186 zu der genannten Stirnseite hin federbelastet. Um den Bügel aus den Nuten 194, 196 heraus verschieben und sodann verschwenken zu können, ist derselbe Lagerzapfen 186 an seinem zur benachbarten Stirnseite der Plattenanordnung 12 hin liegenden Ende mit einem handbetätigten Organ versehen. Beim Ausführungsbeispiel ist letzteres von einem einen Kunststoffmantel 198 aufweisenden Knauf 200 gebildet, der mit einem zum Stützbock 20 hin liegenden, zylindrischen Fortsatz 202 das freie Ende des Lagerzapfens 186 umgibt und daran befestigt ist und der sich bis in eine gegenüber der Bohrung 190 erweiterte, zylindrische Ausnehmung 204 des Schenkels 174 des Stützbocks 20 hinein erstreckt. Zur Federbelastung des Bügels 178 liegt innerhalb der Ausnehmung 204 eine den Lagerzapfen 186 konzentrisch umgebende Schraubendruckfeder 206, die somit gegen Verschmutzung und Berührung geschützt ist. Der Knauf ist für eine den oberhalb von ihm liegenden Querholm 46 tragende Bedienungsperson leicht zugänglich, wozu die bereits beschriebene Abwinklung der Schenkel 50 dieses Querholmes 46 beiträgt; gleiches gilt für die fussseitigen Stützen 22, wo der jeweilige Knauf 200 in nicht näher dargestellter Weise ebenfalls der dort benachbarten Stirnseite der Plattenanordnung 12 zugewandt liegt.

Wie bereits erwähnt, sind die Trageholme 14 unabhängig voneinander höhenverstellbar. Hierdurch ist es möglich, nicht nur bei Bedarf den Kopf oder die Füsse eines Patienten höher zu lagern, sondern auch, den Patienten auf die jeweils zu seiner Versorgung günstigste Höhe zu bringen und auch bei einer Höhenverstellung beispielsweise seiner unteren Extremitäten durch gleichzeitige Tieferstellung seines Kopfes seine mittlere Höhe beizubehalten. Die Höhenverstellung erfolgt zweckmässig mittels hydropneumatischer Betätigungsanordnungen, die beim Ausführungsbeispiel als hydraulische Betätigungsanordnungen ausgebildet sind. Diese seien im folgenden anhand der Fig. 1 und 11 bis 13 näher erläutert.

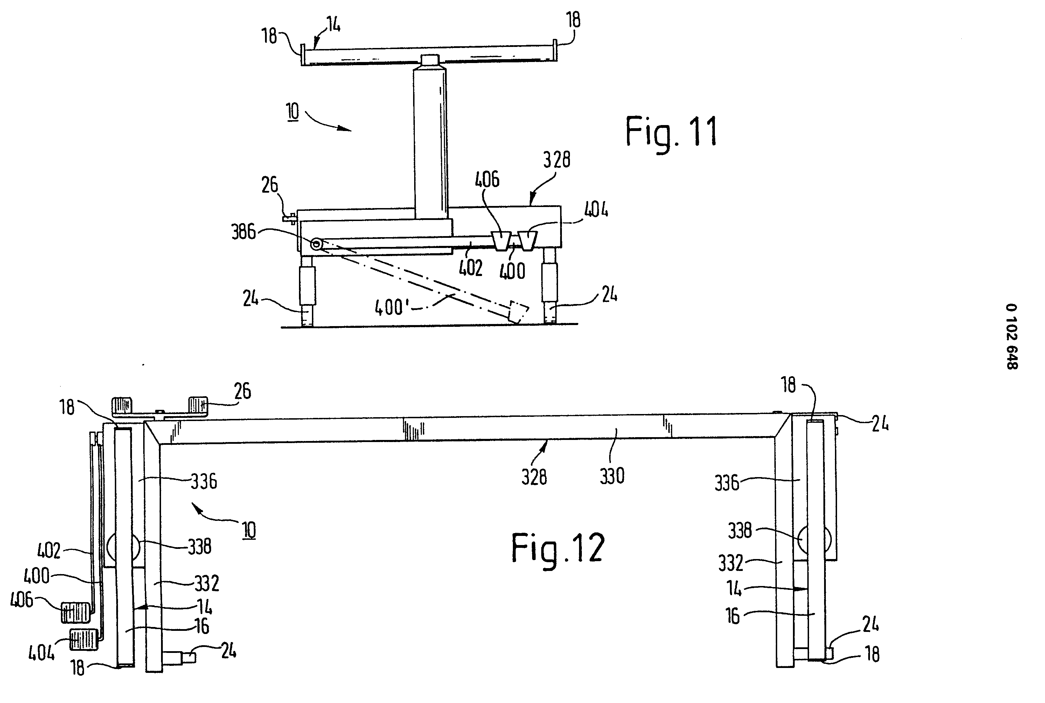

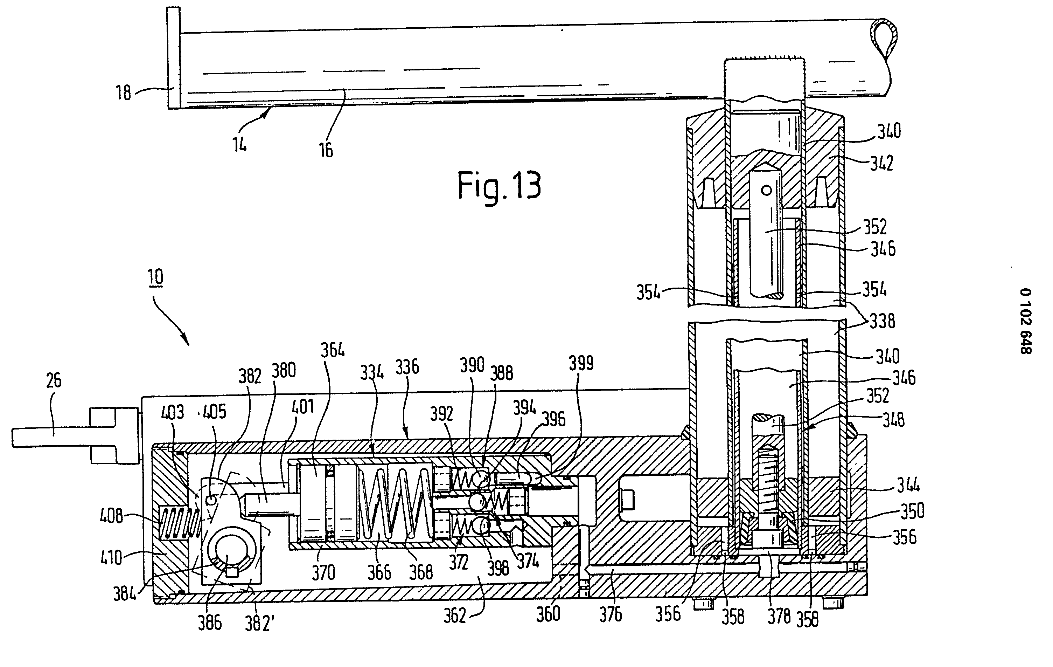

Das Gestell 10 ist auf einer Längsseite offen ausgebildet, um es seitlich über das Unterteil eines Röntgengerätes fahren zu können, über dem dann die Plattenanordnung 12 liegt. Hierzu umfasst es einen in der Draufsicht (Fig. 12) U-förmigen Rahmen 328 mit einem sich entlang einer Längsseite erstreckenden, hohlen Längsträger 330 und zwei sich daran rechtwinklig anschliessenden, sich nahe der Stirnseite erstreckenden Querträgern 332. Die Betätigungsanordnungen umfassen jeweils eine der Druckerzeugung dienende Zylinder-Kolben-Einheit 334 (Fig. 13), im folgenden kurz Druckerzeugereinheit genannt. Die Druckerzeugereinheiten 334 sind jeweils in einem gleichzeitig den Sumpf für die Hydraulikflüssigkeit bildenden, stabilen, allseitig abgedichtet verschlossenen Block 336 untergebracht. Diese sind mit den Querträgern 332 verbunden und erstrecken sich auf deren Aussenseite annähernd von deren am Längsträger 330 liegenden Ende bis über die Mitte des jeweiligen Querträgers 332 hinaus. In den Blöcken 336 ist jeweils in der Längsmittelebene des Gestells 10 ein Ständerrohr 338 gehalten, in dem zentral ein lotrechtes Stützrohr 340 (Fig. 13) verschiebbar und mittels einer oberen Büchse 342 abgedichtet geführt ist. Zwischen dem unteren Ende des Stützrohres 340 und der Innenseite des Ständerrohres 338 ist am Stützrohr 340 ein Stützring 344 gehalten. Am oberen Ende jedes Stützrohres 340 ist ein Trageholm 14 an seiner Mitte befestigt. Innerhalb des Stützrohres liegt ein an seinem unteren Ende abgedichtet gehaltenes Rohr 346, das den Zylinder einer druckbeaufschlagten Zylinder-Kolben-Einheit 348 bildet, die im folgenden kurz als gesteuerte Einheit bezeichnet wird. Der innerhalb des Rohres 346 liegende Kolben 350 der gesteuerten Einheit wirkt über eine zentrale Schubstange 352 auf das obere Ende des Stützrohres 340, so dass eine Verschiebung des Kolbens 350 zugleich eine Höhenverstellung des Stützrohres 340 und des Trageholmes 14 bewirkt. Der Kolben 350 kann bis zum Erreichen von zwei Bohrungen 354 des Stützrohres 340 nach oben ausgefahren werden. Dann fliesst Hydraulikflüssigkeit durch die Bohrungen 354, den Zwischenraum zwischen dem Rohr 346 und dem Stützrohr 340 und teilweise nicht dargestellte Kanäle, darunter Kanäle 356, 358, 360, zurück zu einem den Sumpf der Hydraulikflüssigkeit bildenden Hohlraum 362 des Blocks 336.

Jede Druckerzeugereinheit 334, die im Hohlraum 362 eines Blocks 336 angeordnet ist, umfasst einen Kolben 364, der entgegen der Wirkung einer im Kolbenraum 366 angeordneten Schraubendruckfeder 368 in einen Zylinder 370 eingeschoben werden kann. Bei der entgegengesetzten Ansaugbewegung des Kolbens fliesst Hydraulikflüssigkeit aus dem Hohlraum 362 über ein Rückschlagventil 372 in den Zylinderraum 366. Beim Hineinschieben des Kolbens 364 in den Zylinder 370 gelangt die Hydraulikflüssigkeit über ein dann öffnendes Rückschlagventil 374 und eine Verbindungsleitung 376 in den Zylinderraum 378 der gesteuerten Einheit 348.

Der Stössel 380, über den der Kolben 364 betätigbar ist, wird seinerseits von einer Nocke 382 beaufschlagt, die aus ihrer in Fig. 13 gezeigten Ruhestellung in eine bei 382' angedeutete Stellung verschwenkbar ist. Die Nocke 382 der einen Druckerzeugereinheit 334 ist drehfest verbunden mit einer Hülse 384, die aus dem Block nach aussen abgedichtet herausgeführt ist. Durch die Hülse 384 hindurch verläuft drehbar eine Welle 386, die sich durch den Längsträger 330 hindurch erstreckt und im gegenüberliegenden Block 336 die dort in entsprechender Weise angeordnete Nocke 382 der weiteren Druckerzeugungseinheit 334 betätigt.

Um Hydraulikflüssigkeit aus dem Zylinderraum 378 der gesteuerten Einheit 348 ablassen und damit den Tragoholm 14 nach unten verstellen zu können, ist ein weiteres, gesteuertes Rückschlagventil 388 vorgesehen. Dessen Kugel 390 liegt in einer Bohrung 392, die über eine Drosselbohrung 394 mit der Leitung 376 verbunden ist. Die Kugel 390 kann mittels eines in Richtung auf sie verschiebbaren Fingers 396 von ihrem Ventilsitz abgehoben werden. Der Finger 396 liegt in einem ihm gegenüber weiteren, mit dem Hohlraum 362 verbundenen Kanal 400, durch den bei abgehobener Kugel 390 Druckflüssigkeit sowohl vom Zylinderraum 366 der Druckerzeugereinheit 334 her wie auch über die Drosselbohrung 394 vom Zylinderraum 378 der gesteuerten Einheit 348 her zum Sumpf abströmen kann. Die Drosselbohrung 394 verhindert dabei ein zu schnelles Absenken des Tragoholmes 14.

Der Finger 386 ist am Ende einer Zugstange 402 U-förmig abgebogen, die an ihrem anderen, in Fig. 13 linken Ende mit einer weiteren Abbiegung 404 einen bezüglich der Hülse 384 exzentrisch an der Nocke 382 gebildeten Fortsatz 406 auf dessen der Druckerzeugungseinheit 334 abgewandter Seite hintergreift. Dadurch kann das Rückschlagventil 388 geöffnet werden, indem die Nocke 382 aus ihrer in Fig. 13 ausgezogen dargestellten Ruhestellung im Gegensinn zu einer Betätigung des Kolbens 364, also in Fig. 13 im Gegenuhrzeigersinn, verschwenkt wird. Diese Verschwenkung ist möglich gegen die Wirkung einer Schraubendruckfeder 408, die einerseits an der Nocke 382 abgestützt ist und die andererseits in einem Deckel 410 des Blocks 336 gehalten und abgestützt ist.

Zur Betätigung der Druckerzeugereinheiten 334 sind in den Figuren 1, 11 und 12 dargestellte Fussnebel 400, 402 vorgesehen, die koaxial zueinander nebeneinander gelagert sind und deren Pedale 404, 406 auf im Ruhezustand zumindest annähernd gleicher Höhe nebeneinander angeordnet sind. Der Fusstritthebel 400 ist mit der Hülse 384 (Fig. 13) drehfest verbunden. Der Fusstritthebel 402 ist mit der Welle 386 drehfest verbunden. Jeder der Fusstritthebel 400, 402 betätigt somit nur eine einzige, jeweils einem Tragoholm 14 zugeordnete Betätigungsanordnung. Trotzdem kann durch gleichzeitiges Niedertreten der nebeneinander angeordneten Pedale 404, 406 eine gleichzeitige Höhenverstellung beider Tragoholme 14 erfolgen. Durch Niedertreten nur eines Pedals 404 oder 406 wird nur einer der Tragoholme 14 verstellt. In entsprechender Weise kann durch gleichzeitiges Anheben beider Pedale 404, 406 ein gleichzeitiges Absenken beider Tragoholme 14 erfolgen, während das Anheben nur eines Pedales 404 oder 406 zum Absenken nur eines Tragoholmes 14 führt. Hülse 384 und Welle 386, an denen die Fusstritthebel 400, 402 befestigt und über die sie gelagert sind, befinden sich, wie bereits erwähnt, an dem zum Längsträger 330 hin liegenden Ende eines Querträgers 332. Von dort erstrecken sich die Fusstritthebel 400, 402 in der Ruhestellung in einer waagerechten Richtung zumindest annähernd parallel zu dem genannten Querträger 332. Damit die Fusstritthebel 400, 402 nicht über den ihnen benachbarten Querträger 332 hinaus seitlich aus dem Gestell 10 hervorstehen, wo sie eine Verletzungsgefahr bilden würden, sind sie kürzer als der ihnen benachbarte Querträger 332. Sie sind auch von untereinander verschiedener Länge; der weiter von dem benachbarten Querträger 332 entfernt liegende Fusstritthebel 402 ist kürzer als der näher am benachbarten Querträger 332 liegende Fusstritthebel 400. Die Pedale 404, 406 erstrecken sich senkrecht zu den Fusstritthebeln 400, 402 von dem benachbarten Querträger fort. Sie liegen damit, wie aus Fig. 1 ersichtlich, bei auf dem Gestell 10 getragener Plattenanordnung 12 noch unterhalb von deren einem stirnseitigen Ende und sind damit vor einer unbeabsichtigten Krafteinwirkung geschützt, wie sie sich beispielsweise beim Heranfahren des Tisches an eine Wand ergeben könnten. Trotz der gegenüber dem benachbarten Querträger 332 kürzeren Länge sind die Fusstritthebel 400, 402 noch genügend lang, um bei ihrem Niedertreten bis zum Fussboden einen genügend grossen Schwenkwinkel zu durchlaufen und damit eine schnelle und trotzdem leichte Betätigung der Höhenverstellung der Tragoholme 14 zu gewährleisten; wie aus der in Fig. 11 bei 400' gezeigten, niedergetretenen Stellung des Fusstritthebels 400 ersichtlich, beträgt dessen Schwenkwinkel mehr als 20°.

**Patentansprüche**

1. Tisch zur Lagerung von Patienten, mit einem fahrbaren Gestell (10) und einer darauf angeordneten Plattenanordnung (12), wobei das Gestell (10) nahe seinen Stirnseiten jeweils einen quer zu seiner Längserstreckung verlaufenden, waagerechten Tragoholm (14) mit zwei an dessen Enden befestigten, nach oben ragenden Wangen (18) aufweist, der die Plattenanordnung (12) gegen eine seitliche Verschiebung hält, wobei die Plattenanordnung (12) an den Tragoholmen (14) lösbar gehalten ist und zwei an ihren Längsseiten liegende Längsholme (28) sowie mindestens eine zwischen diesen gehaltene, Röntgenstrahlen

8

durchlässig, ausgebildete Platte (30, 32) aufweist, dadurch gekennzeichnet, dass die Platte aus einer inneren Kunststoffschicht (34, 36) und zwei mit dieser flächig verbundenen, gegenüber der Kunststoffschicht (34, 36) dünnen äusseren Metallschichten (38, 40; 32, 44) besteht.

2. Tisch nach Anspruch 1, dadurch gekennzeichnet, dass die Längsholme (28) an den Stirnseiten der Plattenanordnung (12) mittels Querholmen (46) verbunden sind, die mit den Längsholmen (28) einen rechteckigen Rahmen bilden, und dass die Querholme (46) eine annähernd U-förmige Gestalt mit einem geraden, senkrecht zu den Längsholmen (28) und von der jeweils benachbarten Stirnseite der Platte (30, 32) beabstandet verlaufenden Griffabschnitt (48) und zwei daran zu den Längsholmen (28) abgebogenen und an diesen befestigten Schenkeln (50) aufweisen, die mit in den Längsholmen (28) liegenden und befestigten Zapfen (60) versehen sind.

3. Tisch nach Anspruch 2, dadurch gekennzeichnet, dass jeder Querholm (46) aus einem vorzugsweise rechteckigen Rohr (56) geformt ist und eine Ummantelung (58) vorzugsweise aus einem Kunststoffschaum aufweist, die sich unter Freilassung der Zapfen (60) über den Griffabschnitt (48) und die Schenkel (50) erstreckt und vorzugsweise zumindest teilweise äusserlich fluchtend an einen zumindest annähernd rechteckigen Querschnittsbereich (62) jedes Längsholmes (28) anschliesst, dass die Schenkel (50) einen gegenüber der Ebene der Längsholme (28) von diesen fort nach oben gebogenen Verlauf haben und der Griffabschnitt (48) zumindest teilweise oberhalb der Längsholme (28) liegt und dass die Längsholme (28) einen Hauptquerschnittsbereich (62) umfassen, der eine liegende Unterseite (64), eine an die Unterseite anschliessende, zu ihr senkrechte Aussenseite (66), eine an die Unterseite (64) anschliessende, zu ihr senkrechte, zur Aussenseite (66) parallele und ihr gegenüber weniger hohe erste Innenseite (68), eine an der Aussenseite (66) anschliessende, zur Unterseite parallele erste Oberseite (70), eine an die erste Innenseite (68) anschliessende, zur Unterseite (64) parallele zweite Oberseite (72), eine an diese anschliessend nach oben ragende zweite Innenseite (74) sowie eine Lippe (76) aufweist, die sich ausgehend von der zweiten Innenseite (74) unter Belassung einer Nut (78) von der Dicke einer Platte (30) gleichender Weite beabstandet von der zweiten Oberseite (72) parallel zu dieser erstreckt, wobei in den Nuten (78) der Längsholme (28) die Längsränder (80) einer Platte (30) gehalten sind.

4. Tisch nach einem der vorhergehenden Ansprüche, dadurch gekennzeichnet, dass die Hauptquerschnittsbereiche (62) der Längsholme (28) hohl sind und dass parallel zu den Hauptquerschnittsbereichen (62) und von deren Aussenseiten (66) beabstandet Schienen (86) von stehend-rechteckigem Querschnitt verlaufen, die annähernd in ihrer halben Höhe mit den Hauptquerschnittsbereichen (62) über senkrecht zu ihren Aussenseiten (66) verlaufende Verbindungselemente (88) verbunden sind.

5. Tisch nach Anspruch 3 oder 4, dadurch gekennzeichnet, dass die Längsränder (80) der in den Nuten (78) der Längsholme (28) gehaltenen Platte (30) in den Nuten (78) verklebt sind, dass annähernd über die Länge der in den Nuten (78) der Längsholme (28) gehaltenen Platte (30) an den Innenseiten (68) der Hauptquerschnittsbereiche (62) jeweils ein Schenkel (90) eines Winkelprofils (92) befestigt ist, das mit einem von diesem Schenkel (90) rechtwinklig abgebogenen oberen Schenkel (94) an der Unterseite der genannten Platte (30) anliegt und vorzugsweise durch Verkleben befestigt ist, dass das Winkelprofil (92) einen von seinem oberen Schenkel (94) nach unten abgebogenen Schenkel (96) und einen von dessen unterem Ende von dem benachbarten Längsholm (28) fort und parallel zur Platte (30) einwärts gebogenen unteren Schenkel (98) aufweist, und dass der geringste gegenseitige Abstand der nach unten abgebogenen Schenkel (96) der Winkelprofile (96) der Breite einer von den Stirnseiten der Plattenanordnung (12) her auf die unteren Schenkel (98) einschiebbaren Röntgenfilmkassette (100) gleicht.

6. Tisch nach einem der vorhergehenden Ansprüche, dadurch gekennzeichnet, dass die Plattenanordnung (12) eine zwischen den Längsholmen (28) feststehende Beinplatte (30) und eine Kopfplatte (32) aufweist, die nahe der Beinplatte (30) schwenkbar gegenüber den Längsholmen angelenkt ist und die ausgehend von einer mit der Beinplatte (30) fluchtenden, zwischen den Längsholmen (28) liegenden Lage mit ihrem der Beinplatte (30) abgewandten Kopfende nach oben verschwenkbar und in unterschiedlichen Schwenkwinkeln arretierbar ist, wobei vorzugsweise die Beinplatte (30) und die Kopfplatte (32) gleiche Dicken und gleichen Schichtaufbau aufweisen.

7. Tisch nach Anspruch 6, dadurch gekennzeichnet, dass die Längsholme (28) annähernd über die Länge der Kopfplatte (32) auf ihren Innenseiten (68) jeweils eine Profilleiste (102) tragen, dass an den Profilleisten (102) jeweils eine weitere Leiste (162) befestigt ist, die einen nach unten ragenden Schenkel (166) und einen von dessen unterem Ende von dem benachbarten Längsholm (28) fort und parallel zur Kopfplatte (32) einwärts gebogenen unteren Schenkel (168) aufweist, wobei der geringste gegenseitige Abstand der nach unten abgebogenen Schenkel (166) der Leisten (162) annähernd der Breite einer von den Stirnseiten der Plattenanordnung (12) her auf die unteren Schenkel (168) der Leisten (162) einschiebbaren Röntgenfilmkassette (100) gleicht, und dass der stirnseitige Plattenrand (152) der Kopfplatte (32) eine nach unten offene Ausnehmung hat, deren Oberseite (170) um annähernd die Dicke der Röntgenfilmkassette (100) über den Oberseiten der unteren Schenkel (168) der Leisten (162) liegt und deren Breite mindestens der Breite der Röntgenfilmkassette (100) gleicht.

8. Tisch nach einem der Ansprüche 1 bis 7, dadurch gekennzeichnet, dass an den Unterseiten (64) der Längsholme (28) vier von diesen nach unten ragende Stützblöcke (20, 22) befestigt sind, die jeweils eine in Seitenansicht annähernd umgekehrt U-förmige Gestalt haben und eine nach unten und in Querrichtung offene Ausnehmung (172, 173) aufweisen und von denen jeweils zwei Stützblöcke (20, 22) in einer mit ihren Ausnehmungen (172, 173) einen Trageholm (14) aufnehmenden Stellung auf diesem aufsetzbar sind, und dass die Trageholme (14) zumindest in ihren in den Stützböcken (20, 22) aufnehmbaren Längenabschnitten einen kreisrunden Querschnitt aufweisen und dass die Ausnehmungen (172, 173) der Stützböcke (20, 22) gerundet verlaufen.

9. Tisch nach einem der vorangehenden Ansprüche, dadurch gekennzeichnet, dass die Trageholme (14) unabhängig voneinander höhenverstellbar sind, dass zwei mit demselben Trageholm (14) verrastbare Stützböcke (22) Ausnehmungen (173) aufweisen, deren in Längsrichtung der Plattenanordnung (12) gemessene Weiten grösser sind als die in derselben Richtung gemessenen Querschnittsabmessungen des Trageholmes (14) im Bereich seiner von den genannten Stützböcken (22) beaufschlagten Längenabschnitte, dass zur Höhenverstellung der Trageholme (14) zwei hydropneumatische, vorzugsweise hydraulische Betätigungsanordnungen vorgesehen sind, die jeweils eine der Druckerzeugung dienende Zylinder-Kolben-Einheit (334) und eine druckbeaufschlagte, am Gestell (10) lotrecht angeordnete Zylinder-Kolben-Einheit (348) umfassen und dass die der Druckerzeugung dienenden Zylinder-Kolben-Einheiten (334) mittels Fusstritthebeln (400, 402) betätigbar sind, die koaxial zueinander nebeneinander gelagert sind und deren Pedale (404, 406) in einer Ruhestellung auf zumindest annähernd gleicher Höhe nebeneinander gehalten sind.

10. Tisch nach Anspruch 9, dadurch gekennzeichnet, dass das Gestell (10) einen in der Draufsicht U-förmigen Rahmen (328) mit einem sich entlang einer Längsseite erstreckenden, hohlen Längsträger (330) und zwei sich daran rechtwinklig anschliessenden, sich nahe der Stirnseite erstreckenden Querträgern (332) aufweist, dass die der Druckerzeugung dienenden Zylinder-Kolben-Einheiten (334) jeweils an einem Querträger (332) parallel zu diesem angeordnet sind, dass die Fusstritthebel (400, 402) an dem zum Längsträger (330) hin liegenden Ende eines Querträgers (332) in einer zu diesem zumindest annähernd parallelen Ruhestellung gelagert sind und dass ein Fusstritthebel (402) mittels einer durch den Längsträger (330) hindurchgeführten Welle (386) und der andere Fusstritthebel (400) mittels einer diese Welle (386) umgebenden Büchse (384) jeweils mit der zugeordneten, der Druckerzeugung dienenden Zylinder-Kolben-Einheit (334) in Wirkverbindung steht, dass die Fusstritthebel (400, 402) kürzer als der ihnen benachbarte Querträger (332) und von untereinander verschiedener Länge sind, dass sich die Pedale (404, 406) senkrecht zu ihnen von dem benachbarten Querträger (332) fort erstrecken und dass die Fusstritthebel (400, 402) jeweils bei einem Anheben gegenüber einer Ruhestellung ein Druckentlastungsventil (388) für die zugehörige, druckbeaufschlagte Zylinder-Kolben-Einheit (348) betätigen.

**Revendications**

1. Table pour le couchage de patients, avec un châssis roulant (10) et un ensemble de plaques (12) disposé sur ce châssis, le châssis (10) comportant près de chacun de ses côtés frontaux un longeron porteur horizontal (14) s'étendant transversalement par rapport à la direction longitudinale dudit châssis, avec deux joues (18) fixées à ses extrémités et dépassant vers le haut, qui retient l'ensemble de plaques (12) contre un déplacement latéral, cet ensemble étant fixé de façon détachable aux longerons porteurs (14) et comportant deux longerons longitudinaux (28) disposés au niveau des côtés longitudinaux dudit ensemble et au moins une plaque transparente aux rayons X (30, 32) maintenue entre ces longerons, caractérisée en ce que la plaque est composée d'une couche intérieure de matière plastique (34, 36) et de deux couches métalliques extérieures (38, 40; 32, 44) réunies par une face à cette couche intérieure et minces par rapport à celle-ci.

2. Table selon la revendication 1, caractérisée en ce que les longerons longitudinaux (28) sont réunis aux côtés frontaux de l'ensemble de plaques (12) au moyen de longerons transversaux (46) qui, avec les longerons longitudinaux (28), forment un cadre rectangulaire; et que les longerons transversaux (46) ont une forme approximativement en U, avec une partie de préhension droite, perpendiculaire aux longerons longitudinaux (28) et écartée du côté frontal voisin de la plaque (30, 32), et comportant deux branches (50) pliées par rapport aux longerons longitudinaux (28), fixées à ceux-ci et munies de tenons (60) situés et fixés dans les longerons longitudinaux (28).

3. Table selon la revendication 2, caractéirsée en ce que chaque longeron transversal (46) est formé d'un tube (56), de préférence rectangulaire, et comporte une gaîne (58), de préférence en mousse de matière plastique qui, tout en laissant libres les tenons (60), s'étend sur toute la partie de préhension (48) et les branches (50) et, de préférence, se raccorde en affleurant extérieurement, au moins partiellement, à une partie à section droite rectangulaire, au moins approximativement, (62) de chaque longeron longitudinal (28); que les branches (50) sont pliées vers le haut par rapport au plan des longerons longitudinaux (28); que la partie de préhension (48) est, au moins partiellement, située au-dessus de ces longerons; et que les longerons longitudinaux (28) comprennent une partie à section droite principale (62) qui comporte un côté inférieur (64), un côté extérieur (66) se raccordant au côté inférieur (64) et perpendiculaire à celui-ci, un premier côté intérieur (68) se raccordant au côté inférieur (64), perpendiculaire à celui-ci, parallèle au côté extérieur (66) et moins haut que ce dernier, un premier côté supérieur (70) se raccordant au côté extérieur (66) et parallèle au côté inférieur (64), un deuxième côté supérieur (72) se raccordant au premier côté intérieur (68) et parallèle au côté inférieur (64), un deuxième côté intérieur (74) se raccordant au deuxième côté supérieur (72) et dépassant vers le haut, ainsi qu'une lèvre (76) qui s'étend à partir du deuxième côté intérieur (74) en laissant une rainure (78) de largeur égale à l'épaisseur d'une plaque (30), et qui est écartée du deuxième côté supérieur (72) parallèlement à celui-ci, les bords longitudinaux (80) d'une plaque (30) étant maintenus dans les rainures (78) des longerons longitudinaux (28).

4. Table selon l'une des revendications précédentes, caractérisée en ce que les parties à section droite principale (62) des longerons longitudinaux (28) sont creuses et que, parallèlement à ces parties à section droite principale (62) et écartés de leurs côtés extérieurs (66), s'étendent des rails (86) à section droite rectangulaire verticale et, approximativement à la moitié de leur hauteur sont réunies aux parties à section droite principale (62) par des éléments de liaison (88) s'étendant perpendiculairement à leurs côtés extérieurs (66).

5. Table selon la revendication 3 ou 4, caractérisée en ce que les bords longitudinaux (80) de la plaque (30) maintenue dans les rainures (78) des longerons longitudinaux (28) sont collés dans ces rainures; qu'approximativement sur toute la longueur de la plaque (30) maintenue dans les rainures (78) des longerons longitudinaux (28), une aile (90) d'une cornière (92) est fixée aux côtés intérieurs (68) des parties à section droite principale (62), ladite cornière portant contre le côté inférieur de la plaque (30) par l'intermédiaire d'une aile supérieure (94) pliée à angle droit à partir de cette aile (90) et y étant, de préférence, fixée par collage; que la cornière (92) comporte une aile (96) pliée vers le bas à partir de son aile supérieure (94) et une aile inférieure (98) pliée vers l'intérieur à partir de son extrémité inférieure en s'éloignant du longeron longitudinal voisin (28), parallèlement à la plaque (30); et que la plus petite distance entre les ailes pliées vers le bas (96) des cornières (92) est égale à la largeur d'une cassette à film radiographique (100) qui peut être introduite en la faisant glisser sur l'aile inférieure (98) en partant des côtés frontaux de l'ensemble de plaques (12).

6. Table selon l'une des revendications précédentes, caractérisée en ce que l'ensemble de plaques (12) comporte une plaque de jambes (30) fixe entre les longerons longitudinaux (28) et une plaque de tête (32) qui est articulée près de la plaque de jambes (30) pour pivoter par rapport aux longerons longitudinaux (28) et qui peut être inclinée vers le haut par son extrémité frontale opposée à la plaque de jambes (30) à partir d'une position alignée sur cette plaque de jambes, entre les longerons longitudinaux (28), et peut être bloquée en position à différents angles d'inclinaison, la plaque de jambes (30) et la plaque de tête (32) ayant, de préférence, la même épaisseur et la même structure stratifiée.

7. Table selon la revendication 6, caractérisée en ce que les longerons longitudinaux (28), approximativement sur toute la longueur de la plaque de tête (32), portent chacun sur leur côté intérieur (68) un listeau profilé (102); qu'à chaque listeau profilé (102) est fixé un autre listeau (162) qui comporte une aile dépassant vers le bas (166) et une aile inférieure (168) pliée vers l'intérieur à partir de l'extrémité inférieure de l'aile (166) en s'éloignant du longeron longitudinal voisin (28), parallèlement à la plaque de tête (32), la plus petite distance entre les ailes pliées vers le bas (166) des listeaux (162) étant approximativement égale à la largeur d'une cassette à film radiographique (100) qui peut être introduite en la faisant glisser sur l'aile inférieure (168) des listeaux (162) en partant des côtés frontaux de l'ensemble des plaques (12); et que le bord de plaque (152) du côté frontal de la plaque de tête (32) comporte un évidement ouvert vers le bas, dont le côté supérieur (170) est situé à approximativement l'épaisseur de la cassette à film radiographique (100) au-dessus des côtés supérieurs de l'aile inférieure (168) des listeaux (162) et dont la largeur est au moins égale à la largeur de cette cassette (100).

8. Table selon l'une des revendications précédentes, caractérisée en ce qu'aux côtés inférieurs (64) des longerons longitudinaux (28) sont fixés quatre blocs formant supports dépassant vers le bas (20, 22) qui, dans la vue de côté, ont chacun approximativement une forme un U inversé et comportent un évidement ouvert vers le bas et dans la direction transversale (172, 173), une paire de blocs formant supports (20 ou 22) pouvant être posée sur une traverse porteuse (14) dans une position où les évidements (172, 173) coiffent cette traverse; que les traverses porteuses (14) ont, au moins dans leurs parties longitudinales qui peuvent être reçues dans les blocs formant supports (20, 22), une section circulaire; et que les évidements (172, 173) des blocs formant supports (20, 22) sont arrondis.

9. Table selon l'une des revendications précédentes, caractérisée en ce que les traverses porteuses (14) sont réglables en hauteur indépendamment l'une de l'autre; que deux blocs formant supports (22) encliquetables avec la même traverse porteuse (14) présentent des évidemments (173) dont les longueurs mesurées dans la direction longitudinale de l'ensemble de plaques (12) sont plus grandes que les dimensions de la section droite de la traverse porteuse (14) mesurées dans la même direction dans la zone de ses parties longitudinales sur lesquelles appuient les blocs formant support (22); que, pour le réglage de la hauteur des traverses porteuses (14), deux dispositifs d'actionnement hydropneumatiques, de préférence hydrauliques, sont prévus qui comprennent chacun une unité à cylindre et piston (334) servant à créer une pression et une unité à cylindre et piston soumise à une pression (348) disposée verticalement sur le châssis (10), et que les unités à cylindre et piston qui servent à créer la pression (334) peuvent être actionnées au moyen de leviers à pédale (400, 402) qui tourillonnent dans un palier l'un à côté de l'autre et coaxialement l'un par rapport à l'autre et dont les pédales (404, 406), à une position de repos, sont maintenues l'une à côté de l'autre à la même hauteur, au moins approximativement.

10. Table selon la revendication 9, caractérisée en ce que le châssis (10) comporte un cadre en forme de U en vue de dessus (328), avec un support longitudinal creux (330) s'étendant le long d'un côté longitudinal et deux supports transversaux (332) se raccordant à angle droit à ce support longitudinal et s'étendant près du côté frontal; que les unités à cylindre et piston qui servent à créer une pression (334) sont disposées chacune au niveau d'un support transversal (332), parallèlement à celui-ci; que les leviers à pédale (400, 402) tourillonnent à l'extrémité d'un support transversal (332) située vers le support longitudinal (330), dans une position de repos parallèle, au moins approximativement, à ce support; qu'un levier à pédale (402) est réuni fonctionnellement à l'unité à cylindre et piston (334) associée qui sert à créer une pression, au moyen d'un arbre (386) passé à travers le support longitudinal (330) et l'autre levier à pédale (400) au moyen d'une douille (384) entourant cet arbre (386); que les leviers à pédale (400, 402) sont plus courts que le support transversal voisin (332) et de longueurs différentes entre elles; que les pédales (404, 406) s'étendent perpendiculairement à ces leviers en s'éloignant du support transversal voisin (332); et que les leviers à pédale (400, 402) chaque fois qu'ils sont soulevés par rapport à une position de repos, actionnent une soupape de détente (388) pour l'unité à cylindre et piston sous pression associée (348).

**Claims**

1. A table for supporting patients, with a portable chassis (10) and a plate arrangement (12) disposed thereon, wherein said chassis (10) has near its front ends respectively a horizontal support spar (14) extending transversely to the longitudinal extension of said chassis (10) and having attached to its ends two upwardly extending side pieces (18), and said support spar holds said plate arrangement (12) to prevent a lateral displacement thereof, wherein said plate arrangement (12) is held releasably on said support spars (14) and includes two longitudinal spars (28) located at the longitudinal sides of said plate arrangement (12), as well as at least one plate (30, 32) which is held between said longitudinal spars (28) and allows X-rays to pass through, characterized in that said plate consists of an internal plastic layer (34, 36) and two outer metal layers (38, 40; 32, 44) which are joined to the surfaces of said plastic layer (34, 36) and which are thin in comparison to said plastic layer.

2. Table as claimed in Claim 1, characterized in that said longitudinal spars (28) are connected to each other at the front ends of said plate arrangement (12) by means of transverse spars (46) forming a rectangular frame with said longitudinal spars (28), and that said transverse spars (46) show an approximately U-shaped configuration having a straight grip section (48) extending perpendicularly to said longitudinal spars (28) and being spaced apart from the respectively adjoining front end of said plate (30, 32), and having two legs (50) bent towards said longitudinal spars (28) and attached thereto, said legs (50) being provided with pins (60) positioned and attached within said longitudinal spars (28).

3. Table as claimed in Claim 2, characterized in that each transverse spar (46) is formed by a preferably rectangular tube (56) and comprises a jacket (58), preferably of plastic foam, which extends along said grip section (48) and said legs (50), leaving said pins (60) blank, and joins an at least approximately rectangular cross-sectional area (62) of each longitudinal spar (28), preferably in at least partial external alignment therewith, that said legs (50) are upwardly bent away from said longitudinal spars (28) in relation to the plane of said longitudinal spars, that said grip section (48) is located at least partially above said longitudinal spars (28), and that said longitudinal spars (28) comprise a main cross-sectional area (62) which includes a horizontal bottomside (64), an outerside (66) which is adjacent to said bottomside and perpendicular thereto, a first innerside (68) which is adjacent to said bottomside (64), perpendicular thereto and parallel to and less high than said outerside (66), a first topside (70) which is adjacent to said outerside (66) and parallel to said bottomside, a second topside (72) which is adjacent to said first innerside (68) and parallel to said bottomside (64), a second innerside (74) which is adjacent to said second topside (72) and extends upwardly, as well as a lip (76) which extends from said second topside (72) and leaves a groove (78) having a with corresponding to the thickness of a plate (30), wherein the longitudinal edges (80) of the plate (30) are held in said grooves (78) of said longitudinal spars (28).

4. Table as claimed in any one of the preceding claims, characterized in that said main cross-sectional areas (62) of said longitudinal spars (28) are hollow, and that tracks (86) extend parallel to said main cross-sectional areas (62) and spaced apart from the outersides (66) thereof, said tracks (86) having an upright rectangular cross-section and being connected at approximately half their height with said main cross-sectional areas (62) by means of connection elements (88) extending perpendicularly to said outersides (66) of said main cross-sectional areas

5. Table as claimed in Claim 3 or 4, characterized in that the longitudinal edges (80) of said plate (30), held in the grooves (78) of the said longitudinal spars (28), are glued into said grooves (78), that approximately along the length of said plate (30), held in said grooves (78) of said longitudinal spars (28) respectively, a leg (90) of an angle section (92) is attached to said innersides (68) of said main cross-sectional areas (62), the angle section, with an upper leg (94) bent away from said leg (90) at a right angle, being adjacent to the underside of said plate (30) and preferably attached thereto by glueing, that said angle section (92) has a leg (96) bent downwardly away from said upper leg (94) of said angle section, and a lower leg (98) bent inwardly from the lower end of said leg (96) away from the adjoining longitudinal spar (28) and parallel to said plate (30), and that the smallest distance between said downwardly bent legs (96) of said angle sections (92) corresponds to the width of an X-ray film cassette (100) which can be inserted from the front ends of said plate arrangement (12) onto said lower legs (98).

6. Table as claimed in any one of the preceding claims, characterized in that said plate arrangement (12) has a leg plate (30) fixed between said longitudinal spars (28) and a head plate (32) being close to said leg plate (30) and pivotable relative to said longitudinal spars, said head plate (32), when placed in such a position that it is in alignment with said leg plate (30) and located between said longitudinal spars (28), is upwardly pivotable with its head end averted from said leg plate (30) and can be arrested at various swivel angles, wherein preferably the leg plate (30) and the head plate (32) have the same thickness and the same layer structure.

7. Table as claimed in Claim 6, characterized in that said longitudinal spars (28) carry approximately along the length of said head plate (32) respectively a profile ledge (102) on their innersides (68), that a further ledge (162) is respectively attached to said profile ledge (102), said further ledge comprising a downwardly extending leg (166) and a lower leg (168) bent inwardly from the lower end of said leg (166) away from the adjoining longitudinal spar (28) and parallel to said head plate (32), wherein the smallest distance between said downwardly bent legs (166) of said ledges (162) corresponds approximately to the width of an X-ray film cassette (100) which can be inserted from the front ends of said plate arrangement (12) onto said lower legs (168) of said ledges (162), and that the front edge (152) of said head plate (32) has a downwardly open recess, whose topside (170) is located above the topsides of said lower legs (168) of said ledges (162) at a spacing corresponding approximately to the thickness of said X-ray film cassette (100), and whose width corresponds at least to the width of said X-ray film cassette (100).

8. Table as claimed in any one of Claims 1 to 7, characterized in that, four support blocks (20, 22) are attached to said bottomsides (64) of said longitudinal spars (28), said support blocks (20, 22) extend downwardly from said longitudinal spars and, from a lateral view, respectively have an approximately inverted U-shaped configuration, said support blocks further have a recess (172, 173) open downwardly and in transverse direction, and of said support blocks two support blocks (20, 22) respectively can be placed on a support spar (14) when the support blocks are placed in a position such that their recesses (172, 173) can receive such support spar (14), that the support spars (14) have a circular cross-section at least in their longitudinal sections destinated for reception in said support blocks (20, 22), and that the recesses (172, 173) of said support blocks (20, 22) are rounded.

9. Table as claimed in any one of the preceding claims, characterized in that said support spars (14) can be independently vertically adjusted, that two support blocks (14), which engage with the same support spar (14), have recesses (173) whose widths, measured in longitudinal direction of said plate arrangement (12), are larger than the cross-sectional dimensions of said support spar (14) in the region of its longitudinal sections engaging with said support blocks (22) measured in the same direction, that for vertical adjustment of said support spars (14) there are provided two hydropneumatic, preferably hydraulic actuation systems comprising respectively a cylinder-piston-unit (334) for generating pressure and a pressure-receiving cylinder-piston-unit (348) arranged in a vertical position on the chassis (10), and that said cylinder-piston-units (334) for generating pressure are operated by foot levers (400, 402) mounted coaxially side by side, the pedals (404, 406) of said foot levers (400, 402) in a resting position, being held side by side at at least approximately the same level.

10. Table as claimed in Claim 9, characterized in that said chassis (10) comprises a frame (328) having a U-shaped configuration at plan view, with a hollow longitudinal support (330) extending along one longitudinal side of said frame (328), and two transverse supports (332) connected to said longitudinal support (330) at a right angle and extending close to the front end, that said cylinder-piston-units (334) for generating pressure are respectively arranged on a transverse support (332) in a position parallel thereto, that said foot levers (400, 402) are mounted on the end of a transverse support (332) directed towards said longitudinal support (330), and are, in their inoperative position, at least approximately parallel to said transverse support (332), and that one foot lever (402), by means of a shaft (386) guided within said longitudinal support (330), and the other foot lever (400), by means of a sleeve (384) surrounding said shaft (386), respectively are in operative connection with the associated cylinder-piston-unit (334) for generating pressure, that said foot levers (400, 402) are shorter than said transverse support (332) adjoining thereto and have different lengths, that said pedals (404, 406) extend away from said adjoining transverse support (332) perpendicularly to said foot levers (400, 402), and that said foot levers (400, 402), when lifted out of an inoperative position, respectively actuatea pressure relieve valve (388) for the associated pressure receiving cylinder-piston-unit (348).

Fig.1

Fig.2

0 102 648

Fig.3

Fig.6

Fig. 4

Fig. 5

Fig. 7

Fig. 8

Fig.9

Fig.10

Fig. 11

Fig. 12

Fig.13